# EUROPEAN PATENT SPECIFICATION

(11) **EP 0 960 628 B1**
(45) Date of publication and mention of the grant of the patent: **10.11.2004**
(21) Application number: 96929536.9
(22) Date of filing: 04.09.1996
(51) Int. Cl.: A61M 5/28, A61M 5/24, A61M 5/31

(54) **SYRINGE SERVING ALSO AS AN AMPOULE AND SYRINGE FOR COLLECTING BLOOD**
ALS AMPULLE DIENENDE SPRITZE UND SPRITZE ZUR BLUTENTNAHME
SERINGUE SERVANT EGALEMENT D'AMPOULE ET SERINGUE DE PRELEVEMENT SANGUIN

(30) Priority: 08.09.1995 JP 23126695
(43) Date of publication of application: 01.12.1999
(73) Proprietor: Yoshino, Koichi, Hyogo 659-0016 (JP); Yoshino, Kenichi, Hyogo 651-1141 (JP); Yoshino, Naomi, Hyogo 651-1141 (JP)
(72) Inventor: Yoshino, Koichi, Hyogo 659-0016 (JP)
(74) Representative: Schuhmann, Albrecht
(86) International application number: PCT/JP1996/002522
(87) International publication number: WO 1997/009079

(56) References cited:
- FR-A- 1 478 143
- FR-A- 1 511 044
- FR-A- 2 174 705
- GB-A- 2 181 055
- JP-U- 1 084 651
- JP-U- 55 062 336
- JP-U- 59 048 642
- JP-U- 63 092 636
- US-A- 2 673 561
- US-A- 4 411 656
- US-A- 4 534 758
- US-A- 4 753 638

## Description

### FIELD OF THE INVENTION

The present invention relates to a syringe serving also as an ampule and a syringe for collecting blood, in particular, the syringe having a bellows-like cylindrical portion and a syringe having pleats reinforcement fold by melting formation which make bellows-like cylinder to be elastic smoothly. The present invention relates to a three-way cock which is applicable to above syringe.

### DESCRIPTION OF THE PRIOR ART

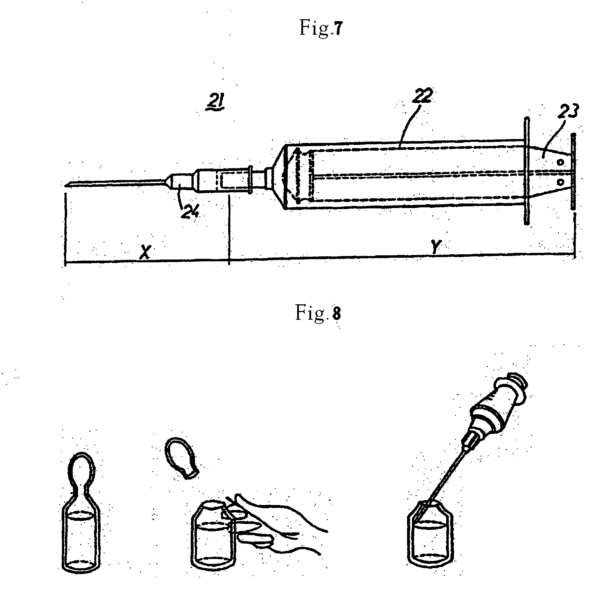

Generally the conventional syringe 21 has the stricture that the piston 23 is inserted within the cylinder 22 and the needle portion 24 is put in the tip shown in Fig.7. In case of injecting a liquid medicine by this conventional syringe, a neck portion of an ampule including the liquid medicine shown in Fig.8 has to be cut with a file so that the needle of the syringe 21 sucks up the liquid medicine in the cylinder of the syringe by inserted within the ampule directly.

In the type of ampule enclosed plastics or cork plug, liquid medicine sucked by inserting the needle of the syringe.

At this time it is troublesome to cut the neck of the above ampule. Furthermore the sharp cut surface of the ampules often hurt fingers if it is made of glass. It is very dangerous for glass ampule to be crushed and inner liquid medicine to be dispersed by earthquake and so on.

About the type of ampule sealed by plug, needle of syringe used to be choked with cork and plastic resin scrap owe to thrusting syringe to sealed cork. It occur to increase infection into body. And it is a problem to give patient a pain with blunt needle which is used to be blunt by piercing through sealed cork. The volume of cork or resin type ampule is big, and twice dividing use yield to be dangerous for infection.

And when various kinds of ampules are put into the syringe, mistakes of picking the ampules are likely to be made. In addition to the danger of hurt with the sharp cut surface, a large amount of empty ampules after use bring the social waste problem.

Same problem has occurred in the waste of conventional syringes. Namely conventional syringes shown in Fig.7 can't be smaller than the length X of the needle portion and the length Y of the cylinder and the piston end portion so that conventional syringes are bulky wastes.

### SUMMARY OF THE INVENTION

The present invention has been made in consideration with the above points at issue the inventor has completed the present invention after a great deal of experiments and improvements to realize the syringe taking little room by uniting the conventional ampule and the syringe which can't be shortened into one.

This invention disclose the syringeserving also as an ampule serves both ampule and syringe which has an extruding piston integrally in a bellows-like body portions with seal film for shuting up the liquid contents, and the blood collecting syringe which has bellows shape syringe, a bellows-contracted-state retainer and a bellows-expanding grip.

They have both being characterised in that the cylindrical portion of the syringe body is made like bellows and that to ensure smooth expansion and/or contraction of the bellows-like cylindrical portion, pleated reinforcing portion are longitudinally provided at several positions by using melting portion. And both syringes has means for holding pressed codition, in the contrary, front and rear wings, are equipped in the syringe and a hook is equipped on the rear wing to hold together. In additionaly, the top end of both syringe is conical and it compose a piston which is a hollow finger pushing portion 9 on the rear portion thereof and a needle portion which can be threadingly fitted to the tip of cylinder body.

The syringe serving also as an ampule has the convex projection inside the needle portion to tear the seal film sealed a content liquid. This syringe serving also as an ampule can be used in drip if the top of syringe shoud be capable to interfit to a three way cock. And in the collecting syringe, back wing which is equipped at the back portion can be used as a holder for stretch bellows.

### BRIEF DESCRIPTION OF THE DRAWINGS

Fig.1(a) is a front view of the body portion of the syringe serving also as an ampule and (b) is a right sectional view of the syringe serving also as an ampule in accordance with the first example of the present invention.
Fig.2 is a front view of the needle portion in accordance with the first example of the present invention. Fig.3 is a perspective illustration of the bellows-like tube portion with the reinforcement gathers in accordance with the second example of the present invention.
Fig.4 is a sectional view of the needle portion in accordance with the second example of the present invention.
Fig.5 is an explanatory view of the syringe for collecting blood in accordance with the forth example of the present invention.
Fig.6 illustrates the syringe serving also as an ampule before wasting in accordance with the first example of the present invention.
Fig.7 is a front view of the conventional syringe.
Fig.8 illustrates the procedure for sucking up the liquid medicine from the conventional ampule.
Fig.9 illustrates the operation at drip infusion in accordance with the third example of the present invention.

### DETAILED DESCRIPTION OF THE PREFERRED EXAMPLE

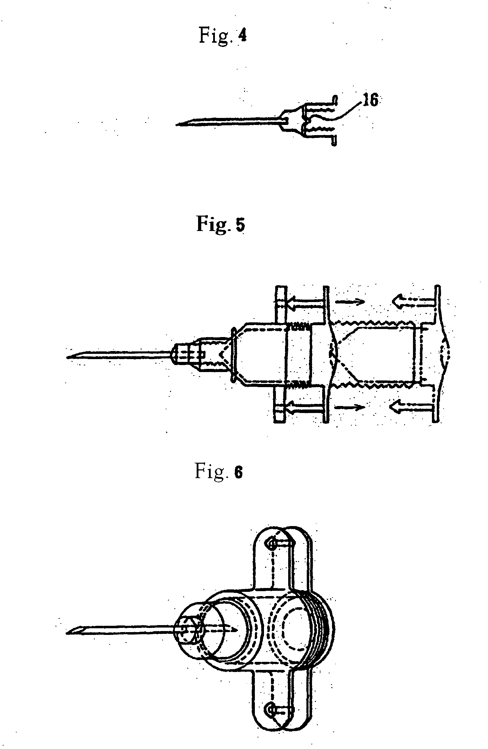

It is desirable to use the medicineproof plastic like fluorine resign, isophthalic acid type unsaturated polyester resin, vinyl ester resin, epoxy resin and fran resin as the material of the syringe serving also as an ampule and the syringe of the present invention. As for the corrosion resistance of resin, it is important effect of curing agent. And the needle for the hypodermic needs to be sharpened on both sides so as to go through the seal film included the liquid medicine. In case of using the projecting needle on one side sharp, it is necessary to set the inner projection 16 shown in Fig.4 to tear the seal film. The projectiong needle on one side is appropriate to the syringe for collecting blood since it doesn't have the seal film.

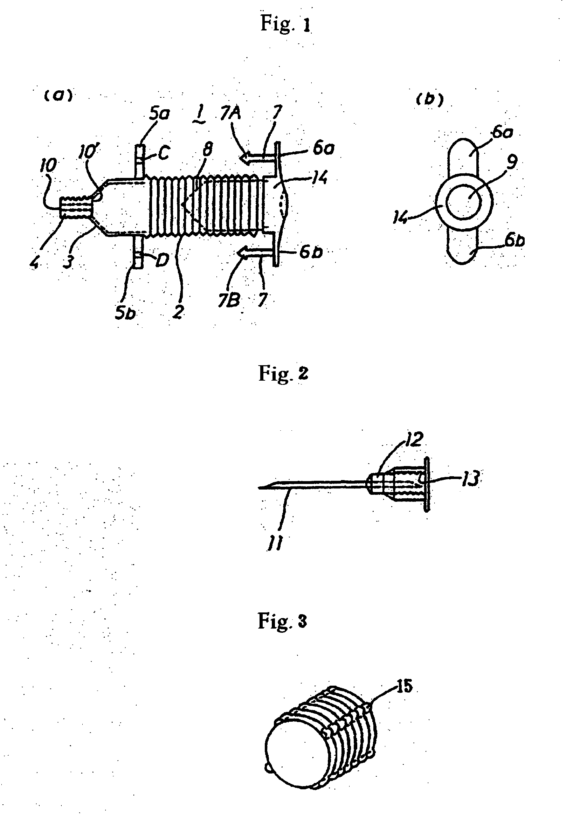

It is necessary that some reinforcement gathers 15 shown in Fig.3 set on the bellows shape tube portion works as pushing pressure passage. The projections and dents of the gathers shown in Fig.3 are shaped to be getting thicker toward outside.

Generally it is an easy method of producing the bellows shape tube portion by moulding each one side half body to join with both sides bodies. At this point of joining, making side thicker than the other portion yield to be two reinforcement gathers on a syringe.

It goes without saying that the number of the reinforcement gathers varies with the size and the use of the syringe.

This reinforcement gathers help not only an syringe but also to prevent bellows bend.

It is possible to join the body portion and the needle portion by moulding the diameter of the body portion to be a little smaller than that of the needle portion. Then concerning the joint pattern, it is also acceptable to thrust the body portion into the needle portion with the screw cutting.

In the case of inserting liquid medicine in drips through three way cock by this invention, the tip of syringe should be made to be threadingly fitted to a three way cock.

The seal film 10, 10' are set in the inner tip of the body portion of the syringe serving also as an ampule to shut up the liquid medicine. It is acceptable for seal film to set at the position 10 or 10' accordance with use method.

And the hollow for pressing by a finger is set at the back of the cylinder.

The projection 7 set on the back wing 6a, 6b are able to be inserted in the inserting hole C, D of the front wing 5a, 5b. And conic head portion 7A, 7B are set at the tip of the projection unless the projection 7 does't come off.

The preferred example of the present invention will be described referring to drawings 1 to 6 and 9.

### [First Example]

This example illustrates the syringe serving also as an ampule for injecting the liquid medicine. Fig.1(a) is a front view of the body portion 1 and (b) is a right sectional view of the syringe serving also as an ampule. 2 is the bellows shape tube portion. 3 is the tip portion. 4 is the screw shape portion. 5a, 5b are the front wing. 6a, 6b are the back wing. 7 is the projection. 8 is the piston. 9 is the hollow for pressing by a finger. 10 is the seal film. Fig.2 is a front view of the needle portion. 11 is the taper shape needle to both sides thereof. 12 is the cap. 13 is the screw shape portion inside the cap.

The body portion 1 made of medicineproof plastic (for example, polypropylene) unitely consists of the tip portion 3, the bellows shape tube portion 2 and the back finger pushing portion 14 with the piston 8. The front wings 5a, 5b are set on the tip portion as well as the back wings 6a, 6b set on the back portion. The back wings 6a, 6b have the projection. The protection has the shaft portion and conic head portion 7A, 7B with the longer diameter than the shaft portion. And the front wings 5a, 5b have the inserting hole C, D with the same diameter as the projecting shaft to fit the projection.

Other type projection can be acceptable in the invention.

The bellows stretches because of including the liquid medicine at the beginning of use with the result that the projection is apart from the inserting hole C, D of the front wing. But after injecting the liquid medicine, the projection 7 is fitted in the inserting hole C, D shown in Fig.6 to cast off. The conic head portion 7A, 7B can't be separated after interfitting because of the shape thereof. The syringe of the present invention is very convenient and safe to scrap compactly.

The seal film 10 set on the most tip portion of the screw shape portion 4 in this example is also able to be set on the inner tip portion 10'.

### [Second Example]

The pleats for reinforcement 15 shown in Fig.3 is vertically attached at 4 points on a bellows-like cylindrical portion according to the first example. The material of the syringe of this present invention is changed with fluorine resin. Only the needle at the leading end is sharpened and inside projection 6(Fig.4) is provided inside the needle portion. Except for them, the syringe is produced in the same way as the first example (Fig. 3).

Thus the pleats do duty for guide against pushing pressure to push the piston, so the syringe of this present invention can be used easily because the pleats for reinforcement are provided.

Accordingly the number of this reinforcement pleats does not matter so much but 2 to 6 pleats are reasonable. And as this example, the convex inside projection 6 breaks through the seal film sealed liquid medicine so the seal film is broken definitely and largely and there isn't in danger of which a broken piece of seal film obstructs a hole inside the needle in comparison with picking by tip of needle

### [Third Example]

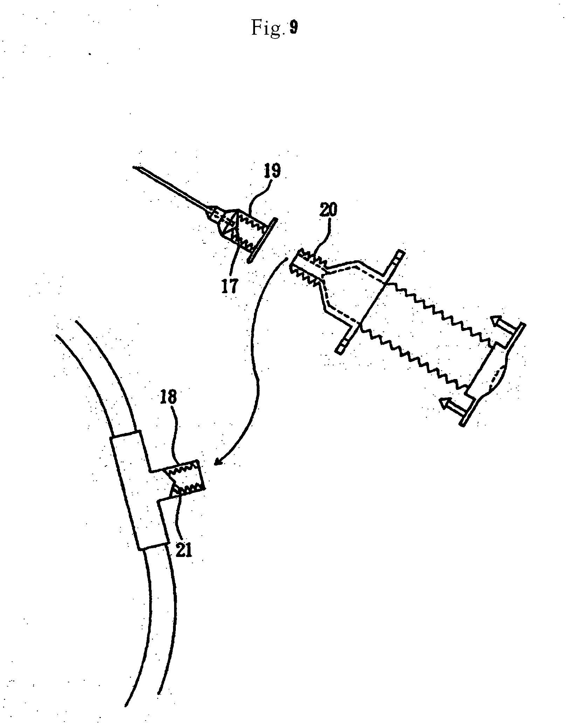

Shown in Fig.9, in this example the material of syringe serving also as ampule is altered to isophthalic acid type unsaturated polyester resin, only a tip of needle is sharpened, inner projection 17(Fig.9) is equipped in the needle portion, the tip of syringe is made for interfitting to three way cock 18 of drip tube.

For adding liquid medicine at drip infusion by this invention, at first seal film 10 is torn by interfitting needle portion 19 to the top end of body 20, and second by taking off needle portion 19 again, the top end of body 20 is interfitted to the three way cock 18. This method of drip style makes it possible to add liquid medicine. To simplyfy the operation for taking off after interfitting needle portion 19 to the top of body 20, and for preventing liquid leak, projection 21 for tearing seal film 10 may be equipped inside three way cock 18.

According to above description, widely use is capable in all medical situation by matching the shape or diameter with faced equippment.

### [Forth Example]

Fig.5 shows a collecting syringe. In this example,needle is oneside sharpend and inside is empty with no liquid medicine, seal film isn't equipped according to example 1. So that the bellows is a contracted state and that will save space at the biginning. In case of collecting blood, the piston is pulled in arrow direction of Fig.5 so the collecting blood is remained in bellows-like cylinder.

The syringe like this is not bulky before collecting blood and after use, for collecting blood may be pulled into syringe by releasing the condition of holding by pulling piston at the direction of arrow in Fig.5 with back wing 6a and front wing as a fulcrum.

This syringe can be disposed with interfitting projection into front wing hole and contracting the bellows as before when collecting blood is transferred in a testtube for check.

Explaining the 1-4 example but it is possible to join the leading end portion of needle and barrel portion by not screw but simple cap setting type. Furthermore it is possible to form integrally the back pressing finger portion 14 and cylinder, also fit into them. But it is desirable to form integrally them in order to inject liquid medicine and keep collecting blood.

### [POSSIBLE APPLICATION IN INDUSTRY]

As describe above, it is no need in this invention of the operation to pull out liquid medicine into syringe and also to cut off the neck of ampule, to dispose ampule in all type syringe as example hypodermic, sarcasm, intramuscular, spread, intravenous, it is impossible to shot at the spot. So the hurt accident is perfectly prevented by ampule cut end. And in collecting syringe it is convenient that the shape before use can be compact. The reinforcement make it smoothly to pull and push operation. And after use it can be contracted and is convenient to dispose.

## Claims

1. A syringe serving also as an ampoule, comprising a partially bellows-like tube (2) having sealed therein a liquid medicine and an extruding piston (8) mounted on one end of said tube, **characterised by** having a film (10) to seal the liquid content at the top end of the syringe body, a needle (11) being sharp at its one end and being attached to a cap (12) at its other end, a projection (16) inside said cap to tear the film (10) inside a needle mounting portion (4) at said top end of the syringe body, a back wing (6a, 6b) with projections (7) on the extruding piston (8) and a front wing (5a, 5b) with holes (C, D) on a front portion of the bellows tube (2) into which holes said projections (7) fit.

2. A syringe as defined in claim 1, **characterised by** having a screw-type top end (20) which is applicable to a three-way drip cock (18) and having the seal film (10) for the liquid contents.

3. A syringe as defined in claim 1 or 2, **characterised in that** the bellows-like tube (2) has thickened pleat reinforcements (15) at points where two moulded half-bodies of the bellows-like portion have been joined together longitudinally by melting.

4. A syringe as defined in any of claims 1-3, **characterised In that** the syringe further comprises a three-way cock (18) comprising a top gate of the syringe body, said cock (18) being threaded on to the syringe and being provided with a projection (21) for tearing the seal film (10).

5. A syringe for collecting blood, said syringe comprising a bellows-like cylindrical tube portion (2); an extruding piston (8) mounted on one end of said tube; a needle (11) being sharp at its one end and being attached to a cap (12) at its other end; means for retaining the bellows in a contracted state (C, D, 7a, 7b); a bellows-expanding grip (6a, 6b); and 2-6 pleated reinforcing portions (15) provided longitudinally on the bellows-like tube (2), said reinforcing portions being thicker at points where two half-bodies of the bellows-like portion have been joined together longitudinally by melting.

## Patentansprüche

1. Spritze, die auch als Ampulle dient, bestehend aus einer teilweise faltenbalgähnlichen Röhre (2), die eine flüssige Medizin einschließt und einem Ausdrückkolben (8), der an einem Ende der Röhre angebracht ist, **gekennzeichnet dadurch, dass** sie eine Folie (10), um den flüssigen Inhalt an der Spitze des Spritzenkörpers zu versiegeln, eine Nadel (11), die an ihrem einen Ende spitz ist und an ihrem anderen Ende an einer Kappe (12) befestigt ist, einen Vorsprung (16) in der Kappe, um die Folie (10) in einem Abschnitt (4) zur Anbringung der Nadel an der Spitze des Spritzenkörpers aufzureißen, einen hinteren Flügel (6a, 6b) mit Vorsprüngen (7) auf dem Ausdrückkolben (8) und einen vorderen Flügel (5a, 5b) mit Löchern (C, D), i die die Vorsprünge (7) passen, an einem vorderen Abschnitt der Faltenbalgröhre (2), besitzt.

2. Spritze gemäß Anspruch 1, **dadurch gekennzeichnet, dass** sie eine Spitze (20) mit einem Gewinde besitzt, die mit einem Dreiwege-Tropfzugang (18) verbindbar ist und die die versiegelnde Folie (10) für den flüssigen Inhalt besitzt.

3. Spritze gemäß Anspruch 1 oder 2, **dadurch gekennzeichnet, dass** die faltenbalgähnliche Röhre (2) verdickte Faltenverstärkungen (15) an Punkten aufweist, an denen zwei gespritzte Halbschalen des faltenbalgähnlichen Abschnitts längs durch Verschmelzen verbunden wurden.

4. Spritze gemäß einem der Ansprüche 1 bis 3, **dadurch gekennzeichnet, dass** die Spritze außerdem einen Dreiwegehahn (18) besitzt, der aus einem vorderen Zugang des Spritzenkörpers besteht, wobei der Hahn (18) auf die Spritze geschraubt ist und einen Vorsprung (21) aufweist, um den versiegelnden Film (10) aufzureißen.

5. Spritze zum Sammeln von Blut, wobei die Spritze aus einem faltenbalgähnlichen, zylindrische Röhrenabschnitt (2); einem Ausdrückkolben (8), der an einem Ende der Röhre angebracht ist; einer Nadel (11), die an einem Ende spitz und an ihrem andere Ende an einer Kappe (12) angebracht ist; Mitteln zum Halten der Falten in einem zusammengezogenen Zustand (C, D, 7a, 7b); einem Griff (&a, &b), um die Falten auseinander zu ziehen; und 2 - 6 faltenverstärkenden Abschnitten (15) die längs der faltenbalgähnlichen Röhre (2) angeordnet sind, wobei die verstärkenden Abschnitte an Punkten dicker sind, an denen zwei Halbschalen des faltenbalgähnlichen Abschnitts längs durch Verschmelzen verbunden wurden.

## Revendications

1. Seringue utilisée aussi comme ampoule, comprenant un tube (2) ayant une forme de soufflet partiel possédant, sous forme scellée à l'intérieur, un liquide médicinal, et un piston (8) d'extrusion monté à une première extrémité du tube, **caractérisée en ce qu'**elle comporte un film (10) destiné à sceller le liquide contenu à l'extrémité supérieure du corps de seringue, une aiguille (11) pointue à une première extrémité et fixée à un capuchon (12) à l'autre extrémité, une saillie (16) placée dans le capuchon et destinée à déchirer le film (10) à l'intérieur d'une partie (4) de montage d'aiguille à l'extrémité supérieure du corps de seringue, une aile arrière (6a, 6b) ayant des saillies (7) sur le piston d'extrusion (8), et une aile avant (5a, 5b) ayant des trous (C, D) à une partie avant du tube en forme de soufflet (2), les saillies (7) s'ajustant dans ces trous.

2. Seringue selon la revendication 1, **caractérisée en ce qu'**elle comporte une extrémité supérieure (20) analogue à une vis qui peut être appliquée sur un robinet (18) de goutte à goutte à trois voies et ayant le film (10) de scellement du liquide contenu.

3. Seringue selon la revendication 1 ou 2, **caractérisée en ce que** le tube (2) en forme de soufflet a des renforcements plissés épaissis (15) à des emplacements auxquels deux demi-corps moulés de la partie en forme de soufflet ont été raccordés longitudinalement par fusion.

4. Seringue selon l'une quelconque des revendications 1 à 3, **caractérisée en ce que** la seringue comporte en outre un robinet à trois voies (18) qui comprend un obturateur supérieur du corps de seringue, le robinet (18) étant vissé sur la seringue et ayant une saillie (21) destinée à déchirer le film (10) de scellement.

5. Seringue destinée à collecter du sang, la seringue comprenant une partie (2) de tube cylindrique en forme de soufflet, un piston d'extrusion (8) monté à une première extrémité du tube, une aiguille (11) qui est pointue à sa première extrémité et qui est fixée à un capuchon (12) à son autre extrémité, un dispositif de retenue du soufflet à l'état contracté (C, D, 7a, 7b), une poignée (6a, 6b) de dilatation de soufflet, et deux à six parties (15) de renforcement plissées disposées longitudinalement sur le tube (2) en forme de soufflet, les parties de renforcement étant plus épaisses à des emplacements auxquels deux demi-corps de la partie en forme de soufflet ont été raccordés longitudinalement par fusion.
